# EUROPÄISCHE PATENTSCHRIFT

(11) **EP 3 349 803 B1**
(45) Veröffentlichungstag und Bekanntmachung des Hinweises auf die Patenterteilung: **07.05.2025**
(21) Anmeldenummer: 16767255.9
(22) Anmeldetag: 16.09.2016
(51) Int. Cl.: A61L 9/12

(54) **RAUMBEDUFTUNGSGERÄT**
AIR FRESHENER DEVICE
DIFFUSEUR DE PARFUM D'AMBIANCE

(30) Priorität: 16.09.2015 EP 15185489
(43) Veröffentlichungstag der Anmeldung: 25.07.2018
(73) Patentinhaber: Good Air AG, 9100 Herisau (CH)
(72) Erfinder: RODERER, Hans, 9100 Herisau (CH); KELLER, Christian, 9403 Goldach (CH)
(74) Vertreter: Hepp Wenger Ryffel AG
(86) Internationale Anmeldenummer: PCT/EP2016/072035
(87) Internationale Veröffentlichungsnummer: WO 2017/046366

(56) Entgegenhaltungen:
- EP-A1- 1 442 754
- EP-A1- 2 564 878
- WO-A1-2006/113254
- WO-A1-2010/132565
- WO-A1-2012/104351
- WO-A1-2013/149285
- WO-A1-2014/145372
- WO-A1-2015/052215
- WO-A2-2013/188499
- DE-A1- 3 243 731
- GB-A- 777 795
- US-A1- 2005 175 513
- US-A1- 2010 314 465

## Beschreibung

Die vorliegende Erfindung betrifft ein Raumbeduftungsgerät.

Um einen Duft in einem Raum abzugeben, sind verschiedene Vorrichtungen und Verfahren bekannt. Eine zentrale Rolle spielt dabei die Art und die Grösse des zu beduftenden Raumes. So werden zur Duftstoffabgabe in Fahrzeuginnenräumen seit vielen Jahrzehnten Einweg-Lufterfrischer eingesetzt. Bei einem solchen Lufterfrischern handelt es sich im einfachsten Fall um vorbehandelte poröse Körper, die nach vollständigem oder teilweisem Entfernen ihrer Verpackung geruchsneutralisierende Substanzen absondern.

Zur Beduftung von grossen Räumen oder ganzen Gebäuden werden dagegen festinstallierte Anlagen eingesetzt. Diese können als Teil der Gebäudelüftung konzipiert sein und den Duftstoff beispielsweise in die Zuluft einleiten. Allerdings können derartige Anlagen auch unabhängig von der Lüftung entworfen sein und beispielsweise aus einem System von Düsen bestehen, durch welche ein Duftstoff direkt in den oder die zu beduftenen Räume versprüht wird.

Einen weiteren Anwendungsbereich stellt die Beduftung von kleinen bis mittelgrossen Räumen, beispielsweise von einzelnen Ladenlokalen, dar. Hierzu werden in aller Regel sogenannte Raumbeduftungsgeräte eingesetzt, die nicht fest installiert sind. Im Stand der Technik sind eine Reihe derartiger Raumbeduftungsgeräte beschrieben.

So bezieht sich die WO 96/31741 auf ein Raumbeduftungsgerät, das in einem kompakten Gehäuse untergebracht ist. Das besagte Gehäuse weist in seinem unteren Bereich einen Lufteinlass und im oberen Bereich einen Luftauslass auf. Zur Bildung eines vertikalen Luftstroms durch das das Gehäuse ist in diesem ein Gebläse angebracht. Der durch das Gebläse erzeugte Luftstrom streicht an einem Verdunstungskörper mit einer Flüssigkeitsverdunstungsfläche vorbei. Innerhalb des Gehäuses ist ferner ein Vorratsbehälter mit einer einen Duftstoff enthaltenden Flüssigkeit angeordnet. Das Ausbringen des Duftstoffes auf die Flüssigkeitsverdunstungsfläche erfolgt über ein spezielles Dosierventil, das von einer Steuerung angesteuert wird.

Bei derartigen Vorrichtungen besteht allerdings das Problem, dass beim Auswechseln oder Befüllen des Vorratsbehälters die Flüssigkeit potentiell auslaufen kann. Dadurch sind solche Geräte für den Heimeinsatz und/oder für die Verwendung durch ungeschultes Personal nur wenig geeignet. Darüber hinaus ist das Dosierventil bei der oben beschriebenen Vorrichtung verhältnismässig komplex ausgestaltet. Ausserdem besteht die Gefahr, dass das Ventil durch nicht flüchtige Anteile in der Duftstoffzusammensetzung verschmutzt oder blockiert wird. Dies macht eine regelmässige Wartung und Reinigung des Dosierventils notwendig. Eine ähnliche Problematik ergibt sich auch in Bezug auf den Verdunstungskörper, auf dessen Verdunstungsfläche sich die nicht flüssigen Restanteile der Duftstoffflüssigkeit zwangsläufig ansammeln. Ein regelmässiges Auswechseln oder Reinigen des Verdunstungskörpers ist daher erforderlich, was unter Umständen nur von fachkundigem Personal bewerkstelligt werden kann. Darüber hinaus ist es bei derartigen Raumbeduftungsgeräten nicht möglich, von einer ersten Duftstoffzusammensetzung auf eine zweite Duftstoffzusammensetzung zu wechseln, ohne das Gerät hierzu zu reinigen oder Verunreinigungen der zweiten Duftstoffzusammensetzung in Kauf zu nehmen.

Das Dokument WO 2010/132565 A1 offenbart einen Flüssigkeitsspender für eine flüchtige Zusammensetzung für die Verwendung in einem Fahrzeug.

Das Dokument WO 2013/149285 A1 offenbart ein Luftreinigungsgerät, welches Keime in einem Luftstrom tötet.

Das Dokument WO 2013/188499 A2 offenbart eine Abgabevorrichtung umfassend ein Gehäuse, ein Gebläse und ein ringförmiges Reservoir.

Das Dokument WO 2014/145372 A1 offenbart eine Vorrichtung mit einem nachfüllbaren oder austauschbaren Behälter zur Abgabe einer geruchskontrollierenden Substanz an eine Umgebung.

Das Dokument WO 2006/113254 A1 offenbart ein nicht-energetisiertes Abgabesystem zur Abgabe oder Freisetzung von flüchtigen Stoffen an die Atmosphäre.

Das Dokument WO 2012/104351 A1 offenbart einen Funktionsknopf für einen Duftstoffbehälter.

Das Dokument WO 2015/052215 A1 offenbart eine Duftstoffabgabevorrichtung für ein Kraftfahrzeug mit einem Luftströmungskanal, der einen Frischlufteinlass, einen Duftstoffauslass und mindestens eine zwischen dem Lufteinlass und dem Auslass angeordnete Öffnung aufweist.

Das Dokument EP 2 564 878 A1 offenbart einen Spender, der in der Lage ist, ein Fluid über eine vertikal ausgerichtete Piezovorrichtung abzugeben.

Das Dokument EP 1 442 754 A1 offenbart eine Vorrichtung zur Dampfabgabe in die Umgebung.

Das Dokument US 2010/314465 A1 offenbart einen Mechanismus zur Veränderung der Umgebungsluft, wie z. B. einen Duftspender.

Das Dokument US 2005/175513 A1 offenbart eine Vorrichtung, die eine Wärmequelle zum Erhitzen und Verteilen von duftenden Ölen oder insektiziden Ölen verwendet.

Das Dokument GB 777 795 A offenbart eine Vorrichtung zur Ausgabe von Substanzen aus einem Vorratsgefäss mit einem Schmierstift.

Das Dokument DE 32 43 731 A1 offenbart eine Vorrichtung zur Ausgabe fliessfähiger Substanzen aus einem Vorratsgefäss.

Es ist daher die Aufgabe der vorliegenden Erfindung, die Nachteile des Standes der Technik zu überwinden.

Insbesondere ist es eine Aufgabe der Erfindung, ein kompaktes Raumbeduftungsgerät für die Beduftung kleiner bis mittelgrosser Räume zu schaffen, das einfach in der Handhabung und zuverlässig im Betrieb ist. Das Gerät soll möglichst mobil, einfach zu transportieren und flexibel einsetzbar sein. Es soll eine effiziente Beduftung ermöglichen, mit einer punktgenauen Steuerung der Duftintensität, die erforderlichenfalls auch möglichst konstant sein soll. Das Raumbeduftungsgerät soll eine einfache Konstruktion aufweisen und unauffällig in einem zu beduftenden Raum platzierbar sein. Allerdings soll das Gerät ferner dazu geeignet sein, in Lüftungssysteme, insbesondere von Fahrzeugen, integriert zu werden.

Diese Aufgaben werden durch ein Raumbeduftungsgerät gelöst, welcher die Merkmale in dem Anspruch 1 aufweist.

Das Raumbeduftungsgerät umfasst einen Luftleitkanal mit einem Lufteinlass und einem Luftauslass, sowie ein Gebläse zum Fördern von Luft durch den Luftleitkanal, vom Lufteinlass zum Luftauslass. Darüber hinaus umfasst das Raumbeduftungsgerät einen Wechselbehälter für eine einen Duftstoff enthaltende Flüssigkeit, sowie eine Dosiereinrichtung für die Flüssigkeit mit einer Dosiereinheit. Ferner umfasst das Raumbeduftungsgerät einen Verdunstungskörper, der innerhalb des Luftleitkanals angeordnet ist, wobei zum Verdunsten der Flüssigkeit diese vom Wechselbehälter mittels der Dosiereinheit zum Verdunstungskörper führbar ist. Die Dosiereinheit ist zumindest teilweise im oder am Wechselbehälter angeordnet und gemeinsam mit diesem wechselbar.

Der Wechselbehälter ist zumindest teilweise aus einem flüssigkeitsundurchlässigen Material, vorzugsweise aus einem Material, das auch gasundurchlässig ist, hergestellt. Bei einem solchen Material kann es sich insbesondere um ein Metall, vorzusweise um eine Aluminiumlegierung handeln.

Dadurch, dass der Wechselbehälter und die Dosiereinheit als ein gemeinsames Wechselmodul ausgeführt sind, entfällt ein Ankoppeln des Behälters an die Dosiereinheit beim Einsetzen in das Gerät. Die Gefahr eines unbeabsichtigten Verschüttens der Flüssigkeit wird daher erheblich reduziert. Ausserdem wird bei jedem Austausch des Wechselbehälters auch die Dosiereinheit ersetzt, womit eine Wartung oder Reinigung desselben entfällt. Dadurch werden ein weitaus zuverlässigerer Betrieb des Raumbeduftungsgerätes und eine einfachere Handhabung desselben erzielt.

Es versteht sich von selbst, dass ein derartiges Raumbeduftungsgerät nicht nur zur Beduftung von Räumen im engeren Sinne geeignet ist, sondern auch zu deren Desodorisierung, namentlich durch die Abgebe von geruchsneutralisierenden Substanzen.

Zusätzlich kann auch der Verdunstungskörper am Wechselbehälter angeordnet und gemeinsam mit diesem wechselbar sein. Dies ist insbesondere von Vorteil, wenn die den Duftstoff enthaltende Flüssigkeit auch nicht-flüchtige Anteile enthält, die während des Betriebs des Raumbeduftungsgerätes auf dem Verdunstungskörper zurückbleiben. Wenn der Verdunstungskörper wie in der besagten Ausführungsform zusammen mit dem Wechselbehälter und der Dosiereinheit ein einheitliches Wechselmodul bilden, entfällt eine Reinigung jeglicher Teile, die mit der Flüssigkeit in Kontakt kommen. Dies ist insbesondere beim Wechsel der Duftstoffzusammensetzung von Vorteil, da eine Kontamination einer zweiten Duftstoffzusammensetzung durch eine zuvor verwendete erste ausgeschlossen werden kann. Dadurch können je nach Bedarf verschiedene Duftstoffzusammensetzungen in verhältnismässig rascher Wechselfolge und wahlweise auch wiederholt mit einem Raumbeduftungsgerät verwendet werden.

Die Dosiereinrichtung kann einen Antrieb umfassen, der über Kopplungsmittel mit der Dosiereinheit koppelbar ist, wobei der Antrieb vorzugsweise im oder am Raumbeduftungsgerät angebracht ist. Durch die Trennung von Dosiereinheit und Antrieb kann der unter Umständen relativ kostspielige und zur wiederholten Verwendung vorgesehene Antrieb als fixe Komponente des Raumbeduftungsgerätes ausgeführt sein. Dadurch wird die Kosteneffizienz des Gerätes bei gleichbleibend hoher Zuverlässigkeit erhöht. Der Antrieb kann ausgewählt sein aus einer Liste bestehend aus einem Hubmagneten, einem Spindelgetriebe, einem Hubkeil, einem Kurbel- oder Nockengetriebe, einem Servomotor, einem Stellmotor und einem Betätigungshebel. Dabei handelt es sich um im gerätebau gebräuchliche Komponenten, die ein zuverlässiges Antreiben der Dosiereinheit ermöglichen.

Die Dosiereinheit umfasst einen Kolben mit einer umlaufenden Nut, wobei der Kolben in einem Zylinder derart verschiebbar gelagert ist, dass die umlaufende Nut über einen Kolbenhub wahlweise mit dem Innenraum des Wechselbehälters oder mit dem Äusseren des Wechselbehälters in Fluidkommunikation bringbar ist. Dies stellt eine besonders einfache und kostengünstige Ausführung der Dosiereinheit dar.

Die Dosiereinheit kann am Boden des Wechselbehälters angebracht sein. Dies hat den Vorteil, dass die gesamte im Wechselbehälter enthaltene Flüssigkeit durch die Dosiereinheit dosierbar ist, ohne dass eine Zuleitung zur Dosiereinheit erforderlich wäre. Die Dosiereinheit kann hierzu zumindest teilweise einstückig mit dem Boden des Wechselbehälters ausgeführt sein. Bevorzugt ist der Boden des Wechselbehälters dabei als separates Teil ausgebildet, während der Deckel des Wechselbehälters insbesondere einstückig mit demselben gebildet ist.

Bei der oben beschriebenen Ausführung der Dosiereinheit kann die umlaufende Nut am oberen Totpunkt des Kolbens mit dem Innenraum des Wechselbehälters und am unteren Totpunkt des Kolbens mit dessen Äusseren in Fluidkommunikation stehen. Insbesondere bei dieser Ausführung, aber auch im Allgemeinen, kann der Kolben durch den Einfluss der Gravitation von seinem oberen Totpunkt zu seinem unteren Totpunkt verschiebbar sein. Allerdings kann der Kolben auch über ein Federelement von einer ausgelenkten Stellung, insbesondre von seinem oberen Totpunkt, in eine Ausgangsstellung, insbesondre zu seinem unteren Totpunkt, rückführbar sein. Sämtliche dieser Varianten ermöglichen es, die Dosiereinheit konstruktiv einfach zu gestalten, wodurch diese kostengünstig hergestellt werden kann und zuverlässig im Betrieb ist.

Bei einer Ausführung der Dosiereinheit mit Federelement kann zusätzlich ein Dämpfungsmittel vorhanden sein. Dieses dient dazu, ruck- oder schlagartige Bewegungen des Kolbens zu verhindern, oder zumindest zu reduzieren. Das Dämpfungsmittel kann einen in einem Zylinder angeordneten, von einer Flüssigkeit um- und/oder durchströmbaren Kolben umfassen. Dabei kann es sich um denselben Kolben und denselben Zylinder handeln, die für die Dosierung der Flüssigkeit verwendet werden. Bei der besagten Flüssigkeit handelt es sich vorzugsweise um die den Duftstoff enthaltende Flüssigkeit im Wechselbehälter. Dies hat den Vorteil, dass nach Aufbrauchen der Flüssigkeit die Dämpfungswirkung reduziert wird bzw. ganz entfällt. Dadurch wird insbesondere durch die veränderten und fallweise auch lauteren Betriebsgeräusche des Gerätes ein Benutzer darauf aufmerksam gemacht, dass die Flüssigkeit aufgebraucht ist.

Das Wechselmodul kann, insbesondere vor dessen erstmaligem Gebrauch, mit einem Sicherungselement gesichert sein. Bei der oben beschreiben Ausführung des Wechselmoduls handelt es sich beim Sicherungselement vorzugsweise um ein gabelartiges Element, das in eine umlaufende Sicherungsnut am Kolben der Dosiereinheit eingreift. Das Sicherungselement verhindert ein versehentliches Betätigen der Dosiereinheit durch blockieren der Längsbewegung des Kolbens im Zylinder.

Bei bestimmungsmässigem Betrieb kann die Flüssigkeit durch den Einfluss der Gravitation zum Verdunstungskörper führbar sein. Dies macht zusätzliche Pump- oder Fördermittel überflüssig.

Der Verdunstungskörper kann in Form einer Membran ausgestaltet sein, die vorzugsweise über einen umlaufenden Rahmen gespannt ist. Die Verwendung einer Membran hat den Vorteil, dass eine solche im Vergleich zu ihrem Volumen eine verhältnismässig grosse Oberfläche aufweist, die gut mit dem Luftstrom in Kontakt gebracht werden kann. Insbesondere, wenn die Verdunstung auf beiden Seiten der Membran erfolgt, kann so eine hohe Verdunstungsrate erzielt werden.

Die Membran kann bei bestimmungsgemässen Betrieb des Raumbeduftungsgerätes horizontal und insbesondere parallel zu dem vom Gebläse erzeugten Luftstrom ausgerichtet sein. Dadurch wird eine optimale Anströmung der Membran mit dem vom Gebläse erzeugten Luftstrom erzielt.

Die Membran kann aus einem Textil, einer Kunststofffolie, einem Vlies oder einem Papier bestehen. Dabei handelt es sich um Materialien, welche die zu verdunstende Flüssigkeit zwar aufnehmen, aber dennoch eine effiziente Abgabe ihrer flüchtigen Anteile an die Umgebungsluft gewährleisten.

Die Membran kann aus einem Funktionstextil mit einer Flüssigkeit speichernden Seite und einer Flüssigkeit abführenden Seite bestehen. Dadurch kann die Verdunstungscharakeristik der Membran zusätzlich verbessert werden.

Bei einem Raumbeduftungsgerät mit einem Antrieb und mit einem als Membran ausgeführten Verdunstungskörper kann die Dosiereinheit durch beidseitige Beaufschlagung der Membran mit dem Antrieb koppelbar sein. Eine auf einer ersten Seite der Membran angebrachte Dosiereinheit wird dabei durch ein zumindest teilweise auf der zweiten Seite der Membran angebrachtes Kopplungsmittel angetrieben, ohne dass ein Durchbruch in der Membran geschaffen werden müsste. Die Membran muss hierzu derart flexibel ausgelegt sein, dass sie eine Hubbewegung des Kopplungsmittels zur Betätigung der Dosiereinheit durch anheben der Membran ermöglicht wird.

Der Luftauslass des Luftleitkanals kann bei bestimmungsgemässen Betrieb in vertikaler Richtung nach oben gerichtet sein. Dadurch kann eine optimale Verteilung der beduftenden Luft in einem Raum erzielt werden.

Das Raumbeduftungsgerät kann eine Halterung für den Wechselbehälter umfassen, wobei der Wechselbehälter vorzugsweise nur in einer Winkelposition in die Halterung einsetzbar ist. Dadurch wird eine zuverlässige und benutzerfreundliche Kopplung der Dosiereinheit mit dem Antrieb gewährleistet. Darüber hinaus wird durch die Halterung sichergestellt, dass der Verdunstungskörper, insbesondere die Membran, stets korrekt im Luftstrom ausgerichtet ist.

Das Einsetzen des Wechselbehälters in die Halterung kann bei bestimmungsmässigem Gebrauch des Raumbeduftungsgerätes in vertikaler Richtung, von oben nach unten erfolgen. Dies hat den Vorteil, dass der Wechselbehälter nicht arretiert werden muss, wenn er sich in der Halterung befindet, und dort einzig durch den Einfluss der Schwerkraft verbleibt. Bei einer solchen Ausführung kann die Kopplung der Dosiereinrichtung mit dem Antrieb über die Kopplungsmittel in gegenläufiger Richtung, vertikal von unten nach oben, erfolgen.

Das Raumbeduftungsgerät kann einen Luftfilter umfassen. Der Luftfilter kann an einem vor dem Gebläse angeordneten Filterrahmen angebracht sein. Das Vorhandensein eines Luftfilters ist insbesondere von Vorteil, wenn das Raumbeduftungsgerät direkt auf dem Fussboden eines zu beduftenden Raumes aufgestellt wird, wo die Staubkonzentration vergleichsweise hoch ist. Durch den Filter wird eine Verschmutzung des Raumbeduftungsgerätes durch die sich am Boden befindenden Staubpartikel verringert. Zudem wird im Wesentlichen verhindert, dass das Gerät Staub aufwirbelt. Ein derartiges mit einem Filter ausgestattetes Gerät kann in gewissen Fällen neben einer Beduftungsfunktion auch eine Reinigungsfunktion der Raumluft wahrnehmen. Vorzugsweise wird bei jedem Wechsel des Wechselbehälters auch der Luftfilter gewechselt.

Ein unbeanspruchter Gegenstand bezieht sich auf einen Wechselbehälter für ein Raumbeduftungsgerät für eine einen Duftstoff enthaltende Flüssigkeit. Dabei ist zumindest teilweise im oder am Wechselbehälter eine Dosiereinheit angeordnet. Ferner kann am Wechselbehälter zusätzlich ein Verdunstungskörper angeordnet sein. Die Dosiereinheit ist dann zur Abgabe der Flüssigkeit auf den Verdunstungskörper mittels eines dem Raumbeduftungsgerät zugeordneten Antriebs betätigbar.

Die Dosiereinheit kann einen Kolben mit einer umlaufenden Nut umfassen. Dabei ist der Kolben in einem Zylinder derart verschiebbar gelagert, dass die umlaufende Nut über einen Kolbenhub wahlweise mit dem Innenraum des Wechselbehälters oder dem Äusseren des Wechselbehälters in Fluidkommunikation bringbar ist.

Der Verdunstungskörper kann in Form einer Membran ausgestaltet sein. Die Membran ist vorzugsweise auf einen umlaufenden Rahmen aufgespannt.

Der Wechselbehälter kann über Mittel zum Druckausgleich mit seiner Umgebung verfügen. Diese Mittel können als ein am Boden des Wechselbehälters angebrachtes Druckausgleichsrohr, als eine in Längsrichtung des Kolbens der Dosiereinheit verlaufende Bohrung, als Beutel oder als Druckausgleichsmembran ausgeführt sein.

Es versteht sich von selbst, dass die oben genannten Einzelmerkmale des Raumbeduftungsgerätes, welche sich auf den Wechselbehälter beziehen, auch bei diesem für sich genommen vorhanden sein können. Ebenso sind einzelne Merkmale des Raumbeduftungsgerätes auch unabhängig vom Wechselbehälter gegenüber dem Stand der Technik besonders vorteilhaft.

Das erfindungsgemässe Raumbeduftungsgerät kann als Einzelgerät zum selbständigen Betrieb oder als Komponente eines Lüftungssystems, insbesondere zum Anschliessen an ein solches, ausgeführt sein.

Ein unbeanspruchter Gegenstand bezieht sich auf ein Lüftungssystem umfassend ein Raumbeduftungsgerät wie oben beschrieben. Das Lüftungssystem kann zumindest einen Frischluftkanal aufweisen. Das Raumbeduftungsgerät kann über einen Bypass an den Frischluftkanal angeschlossen sein. Allerdings kann das Raumbeduftungsgerät auch selbst den Bypass bilden. Es wäre zudem auch denkbar, dass das Raumbeduftungsgerät im Frischluftkanal selbst angeordnet ist.

Weitere Vorteile und Einzelmerkmale der Erfindung ergeben sich aus der nachfolgenden Beschreibung eines Ausführungsbeispiels und aus den Zeichnungen.

Es zeigen schematisch:
Figur 1: Perspektivische Aussenansicht eines erfindungsgemässen Raumbeduftungsgerätes;
Figur 2: Darstellung eines Raumbeduftungsgerätes gemäss Figur 1 ohne die vordere Abdeckung;
Figur 3: Darstellung eines Raumbeduftungsgerätes gemäss Figur **2****,** zusätzlich ohne Innenabdeckung;
Figur 4: Darstellung eines Raumbeduftungsgerätes gemäss Figur **3****,** zusätzlich ohne hintere Abdeckung;
Figur 5: Vereinfachte perspektivische Darstellung einzelner Komponenten eines Raumbeduftungsgerätes gemäss Figur 1 in der zum Betrieb vorgesehenen Anordnung;
Figur 6: Perspektivische Darstellung eines Wechselmoduls für ein Raumbeduftungsgerät gemäss Figur 1;
Figur 7: Detailansicht eines Wechselmoduls gemäss Figur 6 mit Sicherungselement;
Figur 8: Teilweise vereinfachte perspektivische Darstellung einer Gruppe von Einzelkomponenten eines erfindungsgemässen Raumbeduftungsgerätes;
Figur **9****:** Schnittansicht der Darstellung gemäss Figur 8;
Figur 10: Schnittansicht der Darstellung gemäss Figur 8, wobei sich die Gruppe von Einzelkomponenten im betätigten Zustand befindet;
Figur 11: Detailansicht der in Figur 9 erkennbaren Dosiereinheit;
Figuren 12 bis 15: Vereinfachte Prinzipiendarstellung einer Dosiereinheit eines erfindungsgemässen Raumbeduftungsgerätes;
Figur 16: Perspektivische Schnittansicht eines Raumbeduftungsgerätes gemäss Figur 1;
Figur 17: Luftverteilung in einem durch ein erfindungsgemässes Raumbeduftungsgerät bedufteten Raum;
Figuren 18 bis 21: Vereinfachte Prinzipiendarstellungen des Druckausgleiches in einem Wechselbehälter für ein erfindungsgemässes Raumbeduftungsgerät in verschiedenen Varianten;
Figur 22: Eine erste Möglichkeit der Integration eines erfindungsgemässen Raumbeduftungsgerätes in ein Lüftungssystem;
Figur 23: Vergrösserter Teilschnitt eines Raumbeduftungsgerätes gemäss Figur 22;
Figur 24: Eine zweite Möglichkeit der Integration eines erfindungsgemässen Raumbeduftungsgerätes in ein Lüftungssystem.

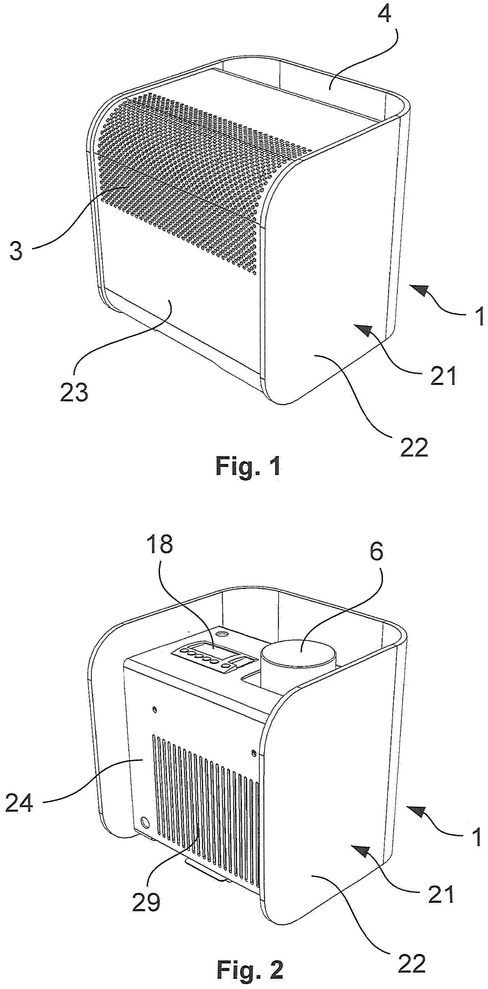

Figur 1 zeigt eine perspektivische Aussenansicht eines erfindungsgemässen Raumbeduftungsgerätes 1. Das Gerät 1 weist ein Aussengehäuse 21 auf, das sich aus einer hinteren Abdeckung 22 und einer vorderen Abdeckung 23 zusammensetzt. Der Lufteinlass 3 wird durch einen Bereich mit einer Vielzahl von Löchern in der vorderen Abdeckung 23 gebildet. Der Luftauslass 4 ist als vertikal nach oben gerichtete Öffnung ausgestaltet. Wie im Folgenden erläutert wird, kommt dieser Ausrichtung des Luftauslasses eine zentrale Bedeutung bei der Verteilung der bedufteten Luft in einem Raum zu.

Wie aus Figur 2 hervorgeht, sind hinter der vorderen Abdeckung 23, welche sich auch über die Oberseite des Gerätes 1 erstreckt, der Wechselbehälter 6 und die Steuereinheit 18 angeordnet. Wenn die vordere Abdeckung am Gerät 1 montiert ist, lässt sich der sich über die Oberseite des Gerätes 1 erstreckende Teil der vorderen Abdeckung 23 entfernen um an den Wechselbehälter 6 und die Steuereinheit 18 zu gelangen. Die beiden Teile 6 und 18 sind damit für einen Benutzer leicht zugänglich, was den Bedienkomfort des Gerätes 1 erheblich erhöht. So kann der Wechselbehälter 6 einfach ausgetauscht werden. Ebenso ist eine bequeme Bedienung der Steuereinheit 18 gewährleistet. Hinter der vorderen Abdeckung 23 ist eine Innenabdeckung 24 angebracht. Die Innenabdeckung 24 weist einen Bereich mit Schlitzen 29 auf, durch den die zu beduftende Luft angesogen wird.

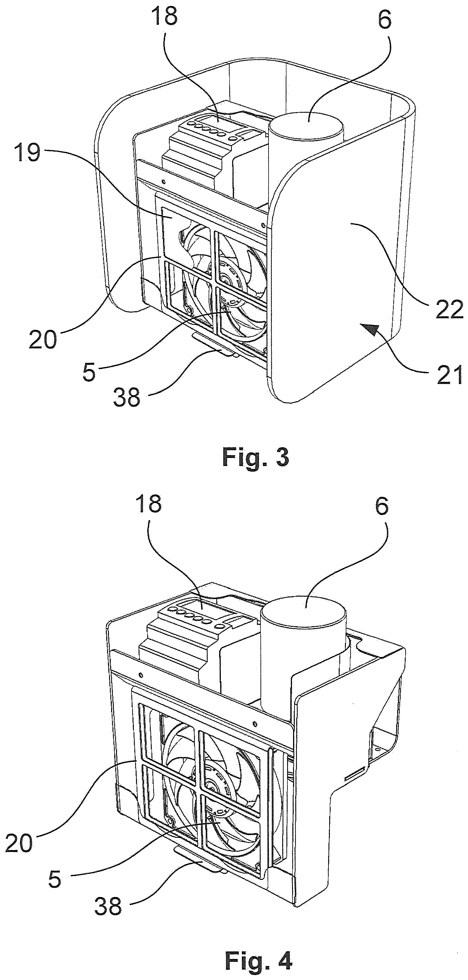

In Figur 3 werden durch das Fehlen der Innenabdeckung 24 weitere Einzelheiten des Raumbeduftungsgerätes 1 ersichtlich. So ist die Steuereinheit 18 nun besser zu erkennen. Darüber hinaus ist das Gebläse 5 sichtbar, welches dazu dient die Luft durch den Luftleitkanal 2 vom Lufteinlass 3 zum Luftauslass 4 zu fördern. Vor dem Gebläse 5 ist ein Filterrahmen 20 angebracht, in welchen ein Luftfilter 19 eingesetzt werden kann, der in der vorliegenden Darstellung nur teilweise dargestellt ist. Der Luftfilter 19 ist mit der Lasche 38 von der Unterseite des Gerätes 1 in den Filterrahmen 20 einsetzbar oder von diesem entnehmbar.

Figur 4 zeigt das Raumbeduftungsgerät 1 gemäss den vorangehenden Figuren 1 bis 3 gänzlich ohne Aussengehäuse 21.

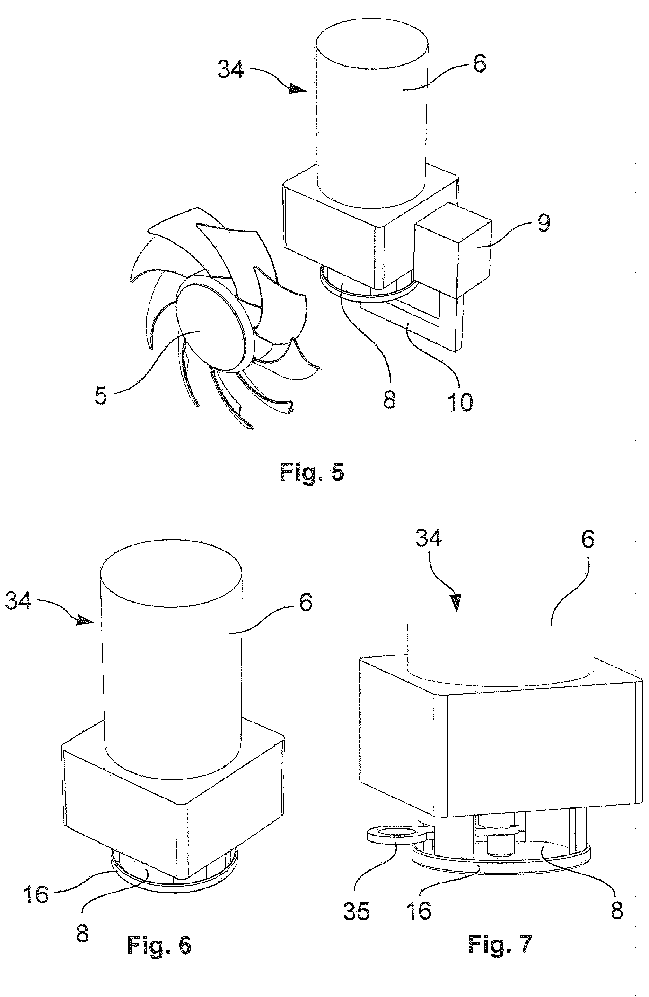

Figur 5 zeigt wesentliche Einzelkomponenten des Raumbeduftungsgerätes 1 gemäss den vorangehenden Figuren 1 bis 4, teilweise vereinfacht, in ihrer relativen Anordnung, wie sie zum Betrieb des Gerätes vorgesehen ist. Es ist zu erkennen, dass das Gebläse 5 derart ausgerichtet ist, dass der von ihm erzeugte Luftstrom beidseitig entlang der Membran 8 streicht. Bei der gezeigten Ausführung bilden der Wechselbehälter 6, die hier nicht sichtbare Dosiereinheit 7 und die Membran 8 ein austauschbares Wechselmodul 34. Der Antrieb 9 für die Dosiereinheit 7 ist über Kopplungsmittel 10 mit derselben koppelbar.

In Figur 6 ist das Wechselmodul 34 bestehend aus Wechselbehälter 6, Dosiereinheit 7 und Membran 8 einzeln zu sehen. Es ist zu erkennen, dass die Membran 8 auf einen kreisrunden umlaufenden Rahmen 16 aufgespannt ist.

Figur 7 zeigt den unteren Bereich des Wechselmoduls 34 vergrössert, wobei das Wechselmodul 34 mit einem Sicherungselement 35 gesichert ist. Beim gezeigteen Sicherungselement 35 handelt es sich um ein gabelartiges Element, das in eine umlaufende Sicherungsnut 39 am Kolben 11 der Dosiereinheit 7 eingreift. Mit dem Sicherungselement 35 kann ein versehentliches Betätigen der Dosiereinheit 7 vor dem erstmaligen Gebrauch des Wechselmoduls 34 verhindert werden.

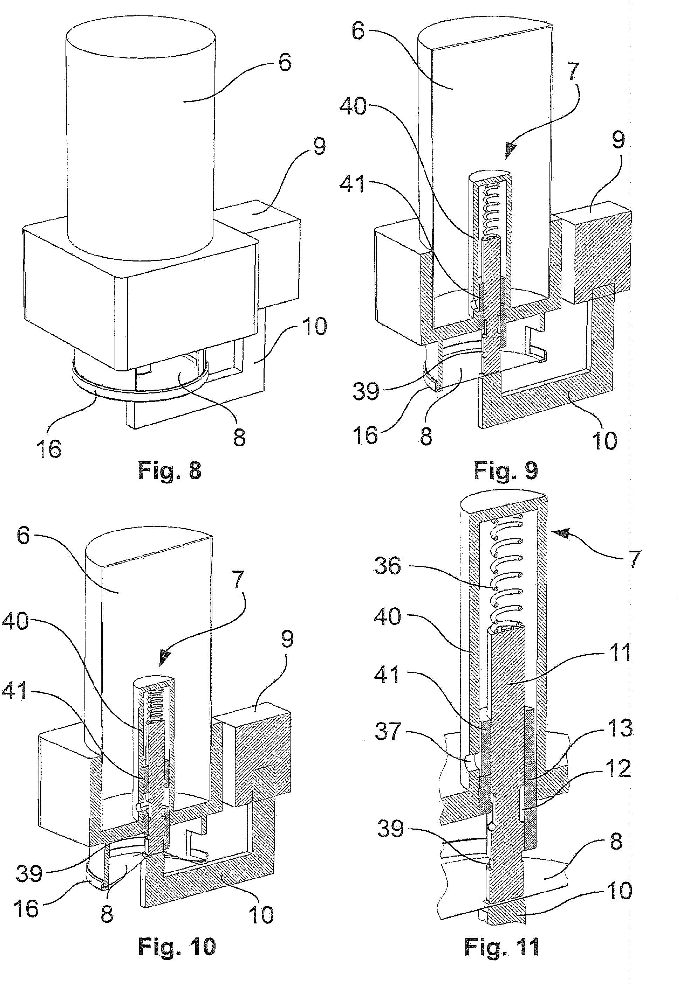

Wie aus den Figuren 8 bis 10 ersichtlich ist, erfolgt bei der gezeigten Ausführungsform die Kopplung der Dosiereinheit 7 mit dem Antrieb 9 über die Kopplungsmittel 10 direkt durch beidseitige Beaufschlagung der Membran 8. Diese wird dabei nicht durchbrochen. Durch die Schnittansichten der Figuren 9 und 10 ist zudem die Anordnung der Dosiereinheit 7 am Boden 15 des Wechselbehälters 6 erkennbar. Figur 9 zeigt die Dosiereinheit 7 im unausgelenkten Zustand, während Figur 10 diese im Ausgelenktem Zustand zeigt. Es ist zu erkennen, dass die Membran 8 durch das Betätigen der Dosiereinheit 7 nach oben gedrückt und zeitweise gedehnt wird.

Aus Figur 11 sind Einzelheiten der Dosiereinheit 7 ersichtlich. Diese umfasst einen Kolben 11 mit einer umlaufenden Nut 12, der in einem Zylinder 13 in Längsrichtung verschiebbar gelagert ist. Der obere Teil des Kolbens 11 reicht in einen zusätzlichen Zylinder 40 hinein. Am Kolben 11 ist ein ringförmiges Element 41 angebracht, das über einen Presssitz fest mit diesem verbunden ist, jedoch mit dem zusätzlichen Zylinder 40 nicht dichtend abschliesst. Durch das Umströmen einer sich im zusätzlichen Zylinder 40 befindenden Flüssigkeit um das ringförmiges Element 41 wird eine Dämpfungswirkung bei einer Hubbewegung des Kolbens 11 erzielt. Im zusätzlichen Zylinder ist eine Feder 36 angebracht, durch welche der Kolben 11 einer Vorspannkraft unterliegt. Der zusätzliche Zylinder 40 weist eine Verbindung 37 zum Innenraum 14 des Wechselbehälters 6 auf. Im unteren Bereich des Kolbens 11 ist ferner die Sicherungsnut 39 zu erkennen.

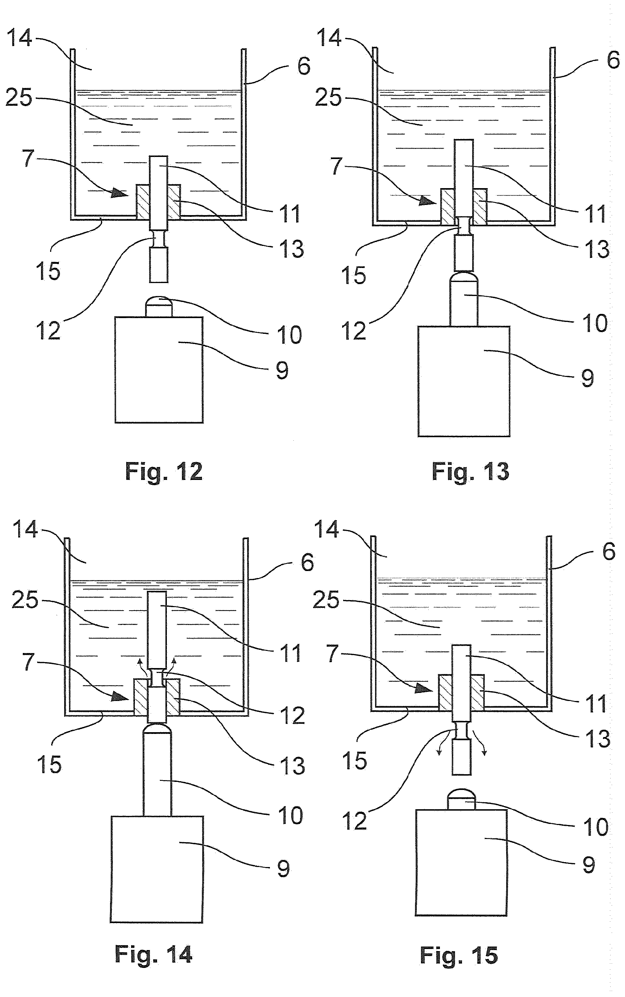

Die Figuren 12 bis 15 verdeutlichen das Funktionsprinzip einer Dosiereinheit 7 eines erfindungsgemässen Raumbeduftungsgerätes **1.** In den gezeigten vereinfachten Darstellungen ist Wechselbehälter 6 ist mit einer einen Duftstoff enthaltenden Flüssigkeit 25 gefüllt, welche die Dosiereinheit 7 umgibt.

In Figur 12 ist die Dosiereinheit 7 mit dem Kolben 11 an seinem unteren Totpunkt dargestellt. Die umlaufende Nut 12 befindet sich dabei in Fluidkommunikation mit dem Äusseren des Wechselbehälters **6.**

In Figur 13 übt der Antrieb 9 über die Kopplungsmittel 10 eine Hubkraft auf den Kolben 11 aus. Dieser wird dadurch nach oben gedrückt.

Figur 14 zeigt die Dosiereinheit 7 mit dem Kolben 11 an seinem oberen Totpunkt. Die umlaufende Nut 12 befindet sich nun in Fluidkommunikation mit dem Innenraum 14 des Wechselbehälters **6.** Sich in der umlaufenden Nut 12 befindende Luft wird entsprechend durch die Flüssigkeit 25 verdrängt und steigt in den Oberteil des Wechselbehälters, wie es durch die beiden Pfeile im Bereich der umlaufenden Nut 12 angedeutet ist.

Figur 15 zeigt die Dosiereinheit 7 nachdem der Kolben 11 wieder in seine Ausgangsposition, namentlich zu seinem unteren Totpunkt, zurückgekehrt ist. Es ist zu erkennen, dass dies im vorliegenden Ausführungsbeispiel selbständig, d.h. einzig durch den Einfluss der Schwerkraft auf den Kolben 11 erfolgt. Am unteren Totpunkt ist die umlaufende Nut 12 wieder in Fluidkommunikation mit dem Äusseren des Wechselbehälters 6, wodurch die sich in der umlaufenden Nut 12 befindende Flüssigkeit nach unten abläuft, wie es durch die beiden Pfeile im Bereich der umlaufenden Nut 12 angedeutet ist.

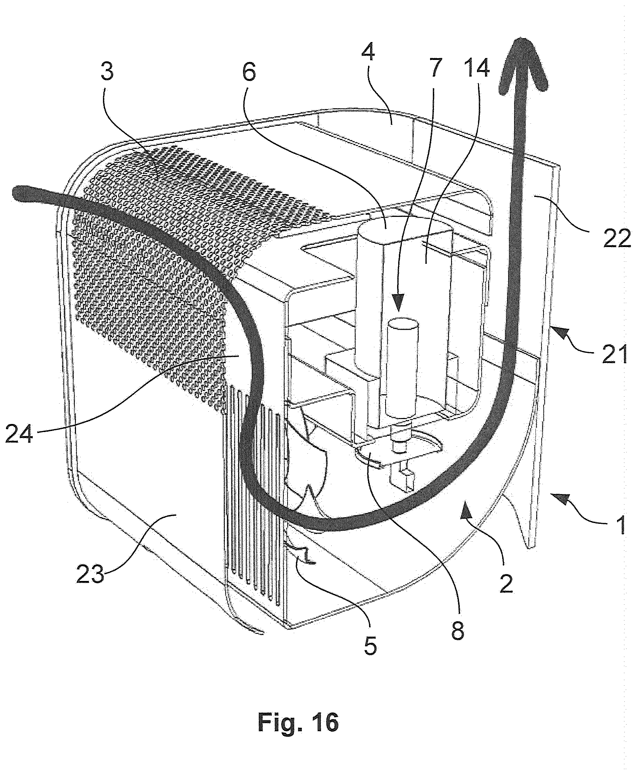

In Figur 16 ist der Luftleitkanal 2 des Raumbeduftungsgerätes 1 besonders gut ersichtlich. Es ist zu erkennen, dass die zu beduftende Luft vom Gebläse 5 durch die vordere Abdeckung 23 und die innere Abdeckung 24 angesogen wird, um danach beidseitig entlang der Membran 8 durch den Luftleitkanal 2 geleitet zu werden. Die die beduftete Luft verlässt das Raumbeduftungsgerät 1 durch den Luftauslass 4 in vertikaler Richtung nach oben.

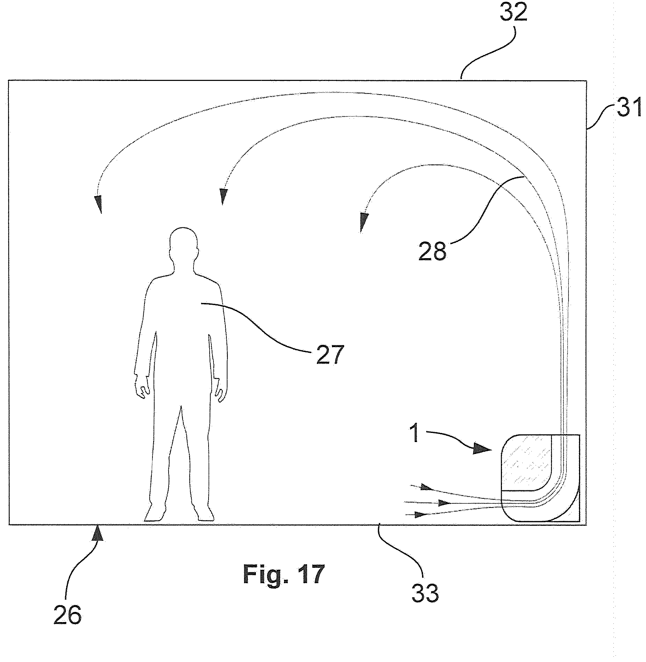

Figur 17 zeigt die Anordnung eines Raumbeduftungsgerätes 1 in einem zu beduftenden Raum 26 mit einer sich darin befindenden Person 27. Ein zentraler Aspekt bei der gezeigten Raumbeduftung besteht darin, dass das Raumbeduftungsgerät 1 im Bereich einer Wand 31 positioniert ist. Die zu beduftende Luft wird in horizontaler Richtung im Bodenbereich des Raumes 26 angesogen und nach erfolgter Beduftung in vertikaler Richtung nach oben entlang der Wand 31 vom Gerät 1 abgegeben. Dadurch bildet die Luft eine Art Konvektionsstrom 28 der sich entlang der Wand 31 und der Raumdecke 32 verteilt. Dies ermöglicht eine besonders vorteilhafte Verteilung der bedufteten Luft im Raum 26, wodurch sich für die Person 27 ein besonders günstiger Dufteindruck ergibt.

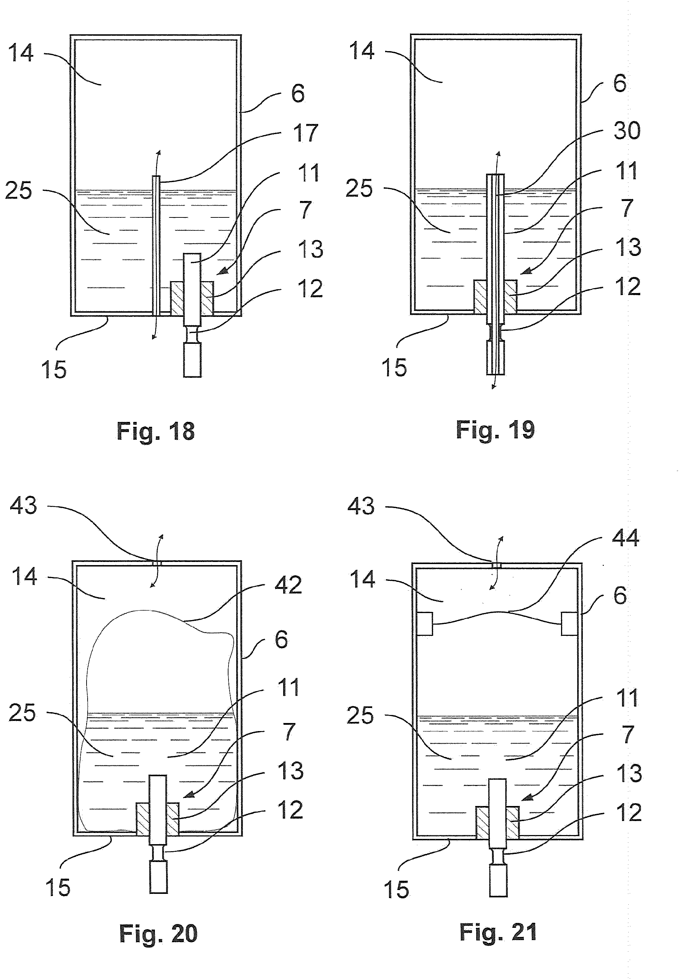

Die Figuren 18 bis 21 zeigen verschiedene Varianten des Druckausgleiches bei einem Wechselbehälter 6 für ein erfindungsgemässes Raumbeduftungsgerät 1. Zwar kommt es durch die Ausgestaltung der Dosiereinheit 7 beim regulären Betrieb des Gerätes 1 grundsätzlich zu einem Druckausgleich im Behälter 6. Trotzdem kann ein zusätzlicher Ausgleich erforderlich sein, insbesondere wenn sich der Umgebungsdruck des Wechselbehälters 6 ändert, ohne dass dieser in Verwendung ist. Dies kann beispielsweise während eines Transportes des Behälters 6 zu einem Verbraucher, oder auch wenn ein erfindungsgemässes Raumbeduftungsgerät 1 in einem Fahrzeug installiert ist, der Fall sein. Druckschwankungen können insbesondere durch eine sich ändernde Meereshöhe oder auch durch Temperaturschwankungen hervorgerufen werden. Auch wenn ein erfindungsgemässes Raumbeduftungsgerät 1 in einem Lüftungssystem installiert ist, kann es zu Druckschwankungen kommen, die einen Druckausgleich im Wechselbehälter 6 erforderlich machen.

Wie aus Figur 18 hervorgeht, kann ein Druckausgleich durch ein am Boden 15 des Wechselbehälters 6 angebrachtes Druckausgleichsrohr 17 herbeigeführt werden. Das Druckausgleichsrohr 17 stellt eine Fluidverbindung zwischen dem Innenraum 14 des Wechselbehälters 6 und seiner Umgebung her (durch Doppelpfeil angedeutet). Aufgrund von Positionierung und Länge des Rohres 17 wird ein Austreten der im Behälter 6 vorhandenen Flüssigkeit 25 unabhängig von dessen Orientierung im Wesentlichen verhindert, so lange der Behälter 6 maximal bis zur Hälfte gefüllt ist. Sollte es dennoch zu einem Austreten von geringen Mengen von Flüssigkeit 25 aus dem Behälter 6 kommen, geraten diese in der Regel auf die Membran 8 (hier nicht gezeigt), wo es lediglich zu einer Verdunstung der Flüssigkeit 25 kommt. Eine Verschmutzung der Umgebung ist damit nahezu ausgeschlossen. Die Figur 19 zeigt eine zu derjenigen gemäss Figur 18 verwandte Ausführung, bei welcher der Druckausgleich über eine in Längsrichtung des Kolbens 11 verlaufende Bohrung 30 erfolgt.

Die Figur 20 zeigt ein alternatives Ausführungsbeispiel eines erfindungsgemässen Wechselbehälters 6 mit Druckausgleich. Hier ist im Innenraum 14 des Behälters 6 ein Beutel 42 angeordnet, in dem die Flüssigkeit 25 enthalten ist. Der Druckausgleich zur Umgebung wird über die Druckausgleichsöffnung 43 hergestellt (durch Doppelpfeil angedeutet). Wird der Beutel 42 durch die Dosiereinheit 7 entleert, kommt es zu einem Kollabieren desselben. Die Figur 21 zeigt eine zu derjenigen gemäss Figur 20 verwandte Ausführung, bei welcher der Druckausgleich über eine im Innenraum 14 des Behälters 6 angebrachte Druckausgleichsmembran 44 erfolgt.

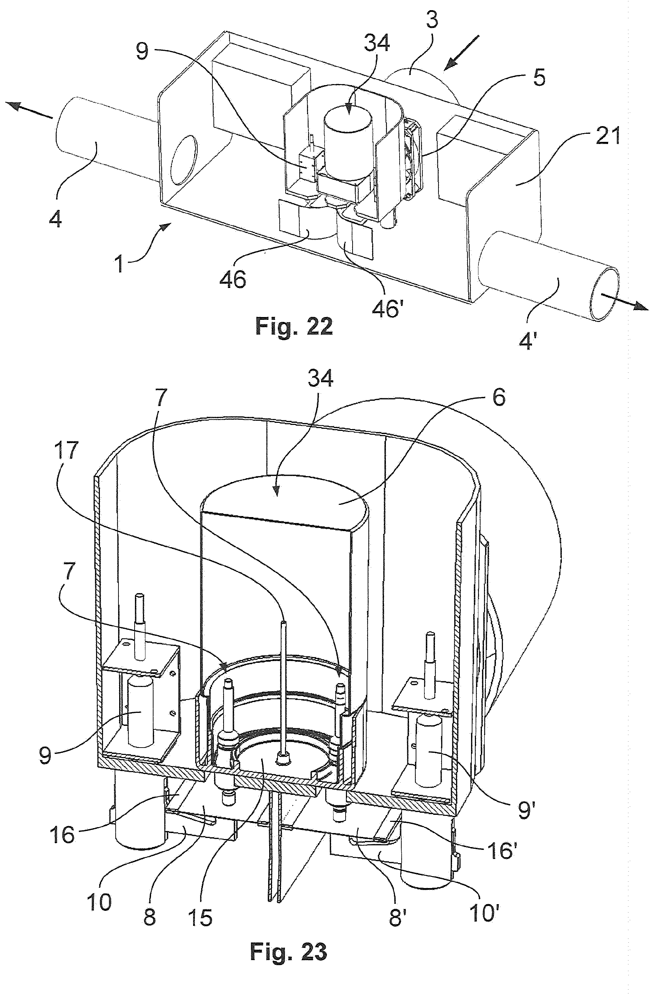

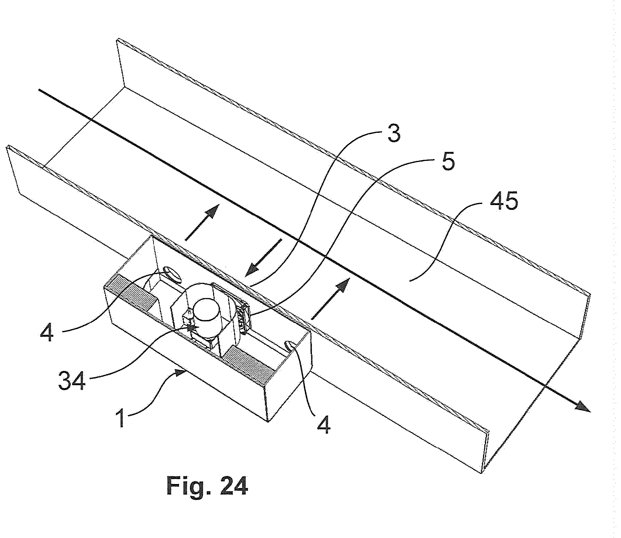

Die Figur 22 zeigt ein weiteres Ausführungsbeispiel eines erfindungsgemässen Raumbeduftungsgerätes 1, welches zum Einbau in ein Lüftungssystem vorgesehen ist. Dabei kann es sich um ein bestehendes Lüftungssystem oder eine Neuinstallation - in einem Gebäude oder in einem Fahrzeug - handeln. Das Gerät 1 wird in der Regel als Bypass an ein luftführendes Element, insbesondere an einen Frischluftkanal 45 (hier nicht gezeigt, siehe jedoch Fig. 24), angeschlossen. Beim besagten Beispiel weist das Gerät 1 einen Lufteinlass 3 und zwei Luftauslasse 4 auf. Der in das Raumbeduftungsgerät 1 eingeleitete Luftstrom wird entsprechend zweigeteilt. Hierzu sind u. a. Luftleitbleche 46, 46' angebracht. Das gezeigte Raumbeduftungsgerät 1 ist dafür vorgesehen, über Rohr- oder Schlauchleitungen an ein Lüftungssystem angekoppelt zu werden.

Wie aus Figur 23 ersichtlich ist, weist das Wechselmodul 34 des Raumbeduftungsgerätes 1 gemäss Figur 22 zwei Dosiereinheiten 7, 7' auf. Auch die Antriebe 9, 9' der Dosiereinheiten 7, 7' sind jeweils in doppelter Ausführung vorhanden. Ferner ist das am Boden 15 des Wechselbehälters 6 angebrachte Druckausgleichsrohr 17 gut zu erkennen.

Die Figur 24 zeigt ein weiteres Ausführungsbeispiel eines erfindungsgemässen Raumbeduftungsgerätes 1, welches zum Betrieb mit einem Lüftungssystem vorgesehen ist. Das Gerät 1 wird an einen Frischluftkanal 45 angebracht, welcher in Längsrichtung von einem Luftstrom durchströmt wird. Das Raumbeduftungsgerät 1 ist über einen Lufteinlass 3 und zwei Luftauslasse 4 mit dem Frischluftkanal 45 verbunden. Im Betrieb wird über das Gebläse 5 Luft aus dem Frischluftkanal 45 angesaugt, bedurftet und wieder in den Frischluftkanal 45 eingeleitet.

## Patentansprüche

1. Raumbeduftungsgerät (1), umfassend einen Luftleitkanal (2) mit einem Lufteinlass (3) und einem Luftauslass (4), ein Gebläse (5) zum Fördern von Luft durch den Luftleitkanal (2) vom Lufteinlass (3) zum Luftauslass (4), einen Wechselbehälter (6) für eine einen Duftstoff enthaltende Flüssigkeit, eine Dosiereinrichtung für die Flüssigkeit mit einer Dosiereinheit (7) und einen Verdunstungskörper (8), der innerhalb des Luftleitkanals (2) angeordnet ist, wobei zum Verdunsten der Flüssigkeit diese vom Wechselbehälter (6) mittels der Dosiereinheit (7) zum Verdunstungskörper (8) führbar ist, wobei die Dosiereinheit (7) zumindest teilweise im oder am Wechselbehälter (6) angeordnet und gemeinsam mit diesem wechselbar ist, **dadurch gekennzeichnet, dass** die Dosiereinheit (7) einen Kolben (11) mit einer umlaufenden Nut (12) umfasst, wobei der Kolben (11) in einem Zylinder (13) derart verschiebbar gelagert ist, dass die umlaufende Nut (12) über einen Kolbenhub wahlweise mit dem Innenraum (14) des Wechselbehälters (6) oder dem Äusseren des Wechselbehälters (6) in Fluidkommunikation bringbar ist.

2. Raumbeduftungsgerät (1) nach Anspruch 1, **dadurch gekennzeichnet, dass** zusätzlich auch der Verdunstungskörper (8) am Wechselbehälter (6) angeordnet und gemeinsam mit diesem wechselbar ist.

3. Raumbeduftungsgerät (1) nach einem der Ansprüche 1 oder 2, **dadurch gekennzeichnet, dass** die Dosiereinrichtung einen Antrieb (9) umfasst, der über Kopplungsmittel (10) mit der Dosiereinheit (7) koppelbar ist und dass der Antrieb (9) vorzugweise im oder am Raumbeduftungsgerät (1) angebracht ist.

4. Raumbeduftungsgerät (1) nach einem der Ansprüche 1 bis 3, **dadurch gekennzeichnet, dass** die umlaufende Nut (12) am oberen Totpunkt des Kolbens (11) mit dem Innenraum (14) des Wechselbehälters (6) und am unteren Totpunkt des Kolbens (11) mit dessen Äusseren in Fluidkommunikation steht.

5. Raumbeduftungsgerät (1) nach einem der Ansprüche 1 bis 4, **dadurch gekennzeichnet, dass** der Kolben (11) durch den Einfluss der Gravitation von seinem oberen Totpunkt zu seinem unteren Totpunkt verschiebbar ist.

6. Raumbeduftungsgerät (1) nach einem der Ansprüche 1 bis 4, **dadurch gekennzeichnet, dass** der Kolben (11) über ein Federelement von seinem oberen Totpunkt, zu seinem unteren Totpunkt, rückführbar ist.

7. Raumbeduftungsgerät (1) nach einem der Ansprüche 1 bis 6, **dadurch gekennzeichnet, dass** bei bestimmungsmässigem Betrieb die Flüssigkeit durch den Einfluss der Gravitation zum Verdunstungskörper (8) führbar ist.

8. Raumbeduftungsgerät (1) nach einem der Ansprüche 1 bis 7, **dadurch gekennzeichnet, dass** der Verdunstungskörper (8) in Form einer Membran ausgestaltet ist, die vorzugsweise auf einen umlaufenden Rahmen (16) aufgespannt ist.

9. Raumbeduftungsgerät (1) nach Anspruch 8, **dadurch gekennzeichnet, dass** die Membran (8) bei bestimmungsmässigem Betrieb des Raumbeduftungsgerätes (1) horizontal und insbesondere parallel zu dem vom Gebläse (5) erzeugten Luftstrom ausgerichtet ist.

10. Raumbeduftungsgerät (1) nach Anspruch 8 oder 9, **dadurch gekennzeichnet, dass** die Membran (8) aus einem Textil, einer Kunststofffolie, einem Vlies oder einem Papier besteht.

11. Raumbeduftungsgerät (1) nach Anspruch 8 oder 9, **dadurch gekennzeichnet, dass** die Membran (8) aus einem Funktionstextil mit einer Flüssigkeit speicherden Seite und einer Flüssigkeit abführenden Seite besteht.

12. Raumbeduftungsgerät (1) nach Anspruch 1 und einem der Ansprüche 8 bis 11, **dadurch gekennzeichnet, dass** die Dosiereinheit (7) durch beidseitige Beaufschlagung der Membran (8) mit dem Antrieb (9) koppelbar ist.

13. Raumbeduftungsgerät (1) nach einem der Ansprüche 1 bis 12, **dadurch gekennzeichnet, dass** der Luftauslass (4) des Luftleitkanals (2) bei bestimmungsmässigem Betrieb in vertikaler Richtung nach oben gerichtet ist.

14. Raumbeduftungsgerät (1) nach einem der Ansprüche 1 bis 13, **dadurch gekennzeichnet, dass** das Raumbeduftungsgerät (1) eine Halterung für den Wechselbehälter (6) umfasst, wobei der Wechselbehälter (6) vorzugsweise in nur einer Winkelposition in die Halterung einsetzbar ist.

15. Raumbeduftungsgerät (1) nach einem der Ansprüche 1 bis 14, **dadurch gekennzeichnet, dass** das Raumbeduftungsgerät (1) eine Steuereinheit (18) für die Dosiereinrichtung umfasst.

## Claims

1. A room fragrancing device (1) comprising an air duct (2) with an air inlet (3) and an air outlet (4), a fan (5) for conveying air through the air duct (2) from the air inlet (3) to the air outlet (4), a replaceable container (6) for a liquid containing a fragrance, a metering device for the liquid with a metering unit (7) and an evaporation body (8) arranged inside the air duct (2), wherein, to evaporate the liquid, it can be fed from the replaceable container (6) to the evaporation body (8) by means of the metering unit (7), wherein the metering unit (7) is arranged at least partially in or on the interchangeable container (6) and can be replaced together with the latter, **characterised in that** the metering unit (7) comprises a piston (11) having a circumferential groove (12), wherein the piston (11) is displaceably mounted in a cylinder (13) in such a way that the peripheral groove (12) can be brought into fluid communication with the interior (14) of the interchangeable container (6) or with the exterior of the interchangeable container (6) as desired over a piston stroke.

2. Room fragrancing device (1) according to claim 1, **characterised in that** the evaporation body (8) is also arranged on the interchangeable container (6) and can be replaced together with the latter.

3. Room fragrancing device (1) according to one of claims 1 or 2, **characterised in that** the metering device comprises a drive (9) which can be coupled to the metering unit (7) via coupling means (10) and that the drive (9) is preferably attached in or on the room fragrancing device (1).

4. Room fragrancing device (1) according to one of the claims 1 to 3, **characterised in that** the circumferential groove (12) is in fluid communication with the interior (14) of the interchangeable container (6) at the top dead centre of the piston (11) and with the exterior of the piston (11) at the bottom dead centre of the piston (11).

5. Room fragrancing device (1) according to one of the claims 1 to 4, **characterised in that** the piston (11) is displaceable from its upper dead centre to its lower dead centre under the influence of gravity.

6. Room fragrancing device (1) according to one of the claims 1 to 4, **characterised in that** the piston (11) can be returned from its upper dead centre to its lower dead centre via a spring element.

7. Room fragrancing device (1) according to one of the claims 1 to 6, **characterised in that** during proper operation the liquid can be guided to the evaporation body (8) under the influence of gravity.

8. Room fragrancing device (1) according to one of the claims 1 to 7, **characterised in that** the evaporation body (8) is configured in the form of a membrane which is preferably stretched on a surrounding frame (16).

9. Room fragrancing device (1) according to claim 8, **characterised in that** the membrane (8) is aligned horizontally and in particular parallel to the air flow generated by the blower (5) when the room fragrancing device (1) is operated as intended.

10. Room fragrancing device (1) according to claim 8 or 9, **characterised in that** the membrane (8) consists of a textile, a plastic film, a fleece or a paper.

11. Room fragrancing device (1) according to claim 8 or 9, **characterised in that** the membrane (8) consists of a functional textile with a liquid-storing side and a liquid-removing side.

12. Room fragrancing device (1) according to claim 1 and one of claims 8 to 11, **characterised in that** the metering unit (7) can be coupled to the drive (9) by acting on both sides of the membrane (8).

13. Room fragrancing device (1) according to one of the claims 1 to 12, **characterised in that** the air outlet (4) of the air duct (2) is directed upwards in a vertical direction when operated as intended.

14. Room fragrancing device (1) according to one of the claims 1 to 13, **characterised in that** the room fragrancing device (1) comprises a holder for the interchangeable container (6), wherein the interchangeable container (6) is preferably insertable into the holder in only one angular position.

15. Room fragrancing device (1) according to one of claims 1 to 14, **characterised in that** the room fragrancing device (1) comprises a control unit (18) for the metering device.

## Revendications

1. Appareil de diffuseur de parfum (1), comprenant un conduit d'air (2) avec une entrée d'air (3) et une sortie d'air (4), un ventilateur (5) pour transporter l'air à travers le conduit d'air (2) de l'entrée d'air (3) à la sortie d'air (4), un réservoir interchangeable (6) pour un liquide contenant un parfum, un dispositif de dosage pour le liquide avec une unité de dosage (7) et un corps d'évaporation (8) qui est disposé à l'intérieur du conduit d'air (2), le liquide pouvant être acheminé du réservoir interchangeable (6) au corps d'évaporation (8) au moyen de l'unité de dosage (7) pour s'évaporer, l'unité de dosage (7) étant disposée au moins en partie dans ou sur le réservoir interchangeable (6) et pouvant être remplacée en même temps que celui-ci, **caractérisé en ce que** l'unité de dosage (7) comprend un piston (11) avec une rainure périphérique (12), le piston (11) étant monté coulissant dans un cylindre (13) de telle sorte de telle sorte que la rainure périphérique (12) peut être mise en communication fluidique, au choix, avec l'intérieur (14) du réservoir interchangeable (6) ou avec l'extérieur du réservoir interchangeable (6) sur une course de piston.

2. Appareil de diffuseur de parfum (1) selon la revendication 1, **caractérisé en ce que** le corps d'évaporation (8) est également disposé sur le réservoir interchangeable (6) et peut être échangé avec celui-ci.

3. Appareil de diffuseur de parfum (1) selon l'une des revendications 1 ou 2, **caractérisé en ce que** le dispositif de dosage comprend un entraînement (9) qui peut être couplé à l'unité de dosage (7) par des moyens de couplage (10) et **en ce que** l'entraînement (9) est de préférence monté dans ou sur l'appareil de diffuseur de parfum (1).

4. Appareil de diffuseur de parfum (1) selon l'une des revendications 1 à 3, **caractérisé en ce que** la rainure périphérique (12) est en communication fluidique avec l'intérieur (14) du réservoir interchangeable (6) au point mort haut du piston (11) et avec l'extérieur de celui-ci au point mort bas du piston (11).

5. Appareil de diffuseur de parfum (1) selon l'une des revendications 1 à 4, **caractérisé en ce que** le piston (11) peut être déplacé de son point mort haut à son point mort bas sous l'influence de la gravité.

6. Appareil de diffuseur de parfum (1) selon l'une des revendications 1 à 4, **caractérisé en ce que** le piston (11) peut être ramené de son point mort haut à son point mort bas par l'intermédiaire d'un élément à ressort.

7. Appareil de diffuseur de parfum (1) selon l'une des revendications 1 à 6, **caractérisé en ce que**, lors d'un fonctionnement conforme à l'usage prévu, le liquide peut être conduit vers le corps d'évaporation (8) sous l'influence de la gravité.

8. Appareil de diffuseur de parfum (1) selon l'une des revendications 1 à 7, **caractérisé en ce que** le corps d'évaporation (8) est conçu sous la forme d'une membrane, qui est de préférence tendue sur un cadre périphérique (16).

9. Appareil de diffuseur de parfum (1) selon la revendication 8, **caractérisé en ce que** la membrane (8) est orientée horizontalement et en particulier parallèlement au flux d'air généré par le ventilateur (5) lorsque l'appareil de diffuseur de parfum (1) fonctionne conformément à sa destination.

10. Appareil de diffuseur de parfum (1) selon la revendication 8 ou 9, **caractérisé en ce que** la membrane (8) est constituée d'un textile, d'un film plastique, d'un non-tissé ou d'un papier.

11. Appareil de diffuseur de parfum (1) selon la revendication 8 ou 9, **caractérisé en ce que** la membrane (8) est constituée d'un textile fonctionnel avec un côté qui stocke le liquide et un côté qui évacue le liquide.

12. Appareil de diffuseur de parfum (1) selon la revendication 1 et l'une des revendications 8 à 11, **caractérisé en ce que** l'unité de dosage (7) peut être couplée à l'entraînement (9) par l'application de la membrane (8) des deux côtés.

13. Appareil de diffuseur de parfum (1) selon l'une des revendications 1 à 12, **caractérisé en ce que** la sortie d'air (4) du conduit d'air (2) est dirigée vers le haut dans le sens vertical en fonctionnement normal.

14. Appareil de diffuseur de parfum (1) selon l'une des revendications 1 à 13, **caractérisé en ce que** l'appareil de diffuseur de parfum (1) comprend un support pour le réservoir interchangeable (6), le réservoir interchangeable (6) pouvant être inséré dans le support de préférence dans une seule position angulaire.

15. Appareil de diffuseur de parfum (1) selon l'une des revendications 1 à 14, **caractérisé en ce que** l'appareil de diffuseur de parfum (1) comprend une unité de commande (18) pour le dispositif de dosage.
